# EUROPÄISCHE PATENTSCHRIFT

(11) **EP 3 496 764 B1**
(45) Veröffentlichungstag und Bekanntmachung des Hinweises auf die Patenterteilung: **17.06.2020**
(21) Anmeldenummer: 17758824.1
(22) Anmeldetag: 11.08.2017
(51) Int. Cl.: C07C 231/12, C07C 269/04, C07C 237/20, C07C 237/36

(54) **BITTERSTOFFDERIVATE**
BITTER PRINCIPLE DERIVATIVES
DÉRIVÉS DE SUBSTANCES AMÈRES

(30) Priorität: 11.08.2016 DE 102016009766
(43) Veröffentlichungstag der Anmeldung: 19.06.2019
(73) Patentinhaber: Julius-Maximilians-Universitaet Wuerzburg, 97070 Wuerzburg (DE)
(72) Erfinder: WYRWA, Ralf, 07715 Rothenstein (DE); RODE, Claudia, 07745 Jena (DE); SEEMANN, Thomas, 07751 Bucha (DE); MEINEL, Lorenz, 97082 Wuerzburg (DE); RITZER, Jennifer, 97230 Estenfeld (DE); SCHNABELRAUCH, Matthias, 07745 Jena (DE)
(74) Vertreter: Teipel, Stephan
(86) Internationale Anmeldenummer: PCT/EP2017/070450
(87) Internationale Veröffentlichungsnummer: WO 2018/029347

(56) Entgegenhaltungen:
- WO-A1-2005/063777
- WO-A1-2013/132058
- GB-A- 1 307 250
- ALFRED SAROLI: "Structure-activity relationship of bitter compounds related to denatonium chloride and dipeptide methyl esters", ZEITSCHRIFT FUER LEBENSMITTELUNTERSUCHUNG UND -FORSCHUNG., Bd. 182, Nr. 2, 1. Februar 1986 (1986-02-01), Seiten 118-120, XP055421926, XX ISSN: 0044-3026, DOI: 10.1007/BF01454242
- DATABASE ZREGISTRY [Online] SERVICE, COLUMBUS, OHIO, US; 16. Mai 2014 (2014-05-16), XP002775339, Database accession no. 1605618-01-6
- DATABASE ZREGISTRY [Online] SERVICE, COLUMBUS, OHIO, US; 5. Mai 2014 (2014-05-05), XP002775340, Database accession no. 1597451-18-7
- DATABASE ZREGISTRY [Online] SERVICE, COLUMBUS, OHIO, US; 1. Mai 2014 (2014-05-01), XP002775341, Database accession no. 1595199-59-9
- DATABASE ZREGISTRY [Online] SERVICE, COLUMBUS, OHIO, US; 1. April 2014 (2014-04-01), XP002775342, Database accession no. 1578182-21-4
- DATABASE ZREGISTRY [Online] SERVICE, COLUMBUS, OHIO, US; 28. März 2014 (2014-03-28), XP002775343, Database accession no. 1575693-30-9

## Beschreibung

### Gebiet der Erfindung

Beschrieben wird die Synthese von Bitterstoffderivaten durch Einführung kupplungsfähiger Gruppen in die Denatoniumstruktur. Die Synthese ermöglicht die Generierung einer Vielzahl an Substitutionsmustern. Die Erfindung dient der Bereitstellung von Bitterstoffderivaten für Anwendungen in der Medizin, Biologie, Medizintechnik sowie in der Kosmetik, der pharmazeutischen, chemischen und Nahrungsmittelindustrie.

### Stand der Technik

Es gibt eine Reihe an natürlichen Bitterstoffen aus den Stoffklassen der Glycoside, Isoprenoide, Alkaloide und Peptide [S. Rodgers et al., Chem. Senses 30, 2005, 547-557; A. Troszyńska, Pol. J. Food Nutr. Sci. 13/54, 2004, 65-73; A. Drewnowski et al., Am. J. Clin. Nutr. 72, 2000, 1424-1435]. Zu diesen Bitterstoffen zählen Naringin, Cucurbitacin, Lactucopikrin, Prämarrubiin, Marrubiin, Cynarin, Lactucin, Coffein, Theobromin, Chinin und Chinchonidin. Bei der bittersten natürlichen Substanz handelt es sich um Amarogentin aus der Enzianwurzel. Auch wurde bisher eine Vielzahl an synthetischen Bitterstoffen hergestellt. Dazu gehören beispielsweise quartäre Ammoniumverbindungen [A. Saroli, Z Lebensm Unters Forsch 182, 1986, 118-120]. Die bitterste Substanz ist dabei Benzyldiethyl(2,6-xylylcarbamoyl)methylammoniumbenzoat, welches auch unter dem Namen Denatoniumbenzoat, Bitrex®, Aversion® geführt wird [EP 0955309 A1]. Der bittere Bestandteil ist dabei das Benzyldiethyl(2,6-xylylcarbamoyl)-methylammonium-Ion. Daher sind auch das kommerzielle Denatoniumsaccharinat oder auch das Capsaicinat [US000005891919A] stark bittere Substanzen. Als Bitterstoff wird Denatonium in unterschiedlichen Anwendungsgebieten verwendet. Denatoniumbenzoat wird beispielsweise zur Vergällung von Ethanol benutzt, so dass sich der Alkohol nicht mehr für den menschlichen Genuss eignet. Andere Alkohole sowie Lösungsmittel, Reinigungsmittel, Shampoos, Seifen werden ebenfalls mit Denatoniumbenzoat versetzt, um gesundheitliche Schäden durch deren orale Aufnahme zu vermeiden. Der Bitterstoff wird auch als Zusatz in Nagellack gegen das Fingernagelkauen verwendet. Da Ratten Denatoniumbenzoat bei geringen Konzentrationen nicht wahrnehmen, ist es auch ein geeigneter Sicherheitszusatzstoff im Rattengift. WO 2013/132058 beschreibt einen diagnostischen Kaugummi zur Identifizierung von Pathogenen durch den Geschmackssinn. In einer Ausführungsform wird ein Geschmacksmolekül über eine Polypeptidkette mit dem Grundmaterial verbunden. Denatonium wird in dem Dokument nicht genannt.

Trotz einer Vielzahl an natürlichen und synthetischen Bitterstoffen ist eine kovalente Anbindung von Bitterstoffen zur Erschließung weiterer Anwendungsgebiete bisher ein ungelöstes Problem. Durch eine Derivatisierung verlieren die Substanzen meist ihren bitteren Geschmack. Zudem ist die Derivatisierung natürlicher Bitterstoffe unwirtschaftlich, weil die Isolierung dieser Substanzen aus Pflanzen einen kostenintensiven Prozess darstellt. Auch ist eine Vielzahl an natürlichen Substanzen für eine chemische Derivatisierung ungeeignet, da entweder geeignete Funktionalitäten fehlen oder die Substanzen während der Synthese zersetzt werden.

Daher besteht ein dringender Bedarf an Bitterstoffen, die eine einfache Derivatisierung mit unterschiedlichen Resten ermöglichen. Dabei soll die Synthese derartiger Substanzen in einem einfachen Verfahren, kostengünstig und in technischen Maßstäben reproduzierbar durchführbar sein, um den steigenden Bedarf aus Medizin, Biologie, Medizintechnik sowie der Kosmetik, der pharmazeutischen, chemischen und Nahrungsmittelindustrie zu decken.

### Zusammenfassung der Erfindung

Die vorliegende Erfindung betrifft:
1. Verbindung der allgemeinen Formel 1 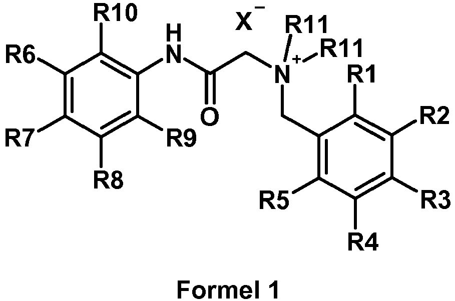 wobei
   X⁻ Halogenid, Pseudohalogenid, Sulfat, Benzoat, Acetat, Trifluoracetat, Hydroxid, Saccharinat oder Capsaicinat bedeutet,
   R1-R10 unabhängig voneinander Wasserstoff, Halogen, C1-C5-Alkyl-, C1-C4-Alkoxy-, C1-C20-Alkoxycarbonyl-, -NH-P, -O-P, wobei P ein Wasserstoff, ein Peptidrest bestehend aus 1 - 30 Aminosäuren, wobei es sich um modifizierte und unmodifizierte D-Aminosäuren, L-Aminosäuren sowie unnatürliche Aminosäuren handeln kann, ist, -(Y)ₙ-COOR13, -(Y)ₙ-COOM mit M = Na, K, [N(R12)₄]⁺ oder -(Y)ₙ-C(O)NR14R15 bedeuten,
   wobei R14 und R15 Wasserstoff, einen C1-C12-Alkyl- oder einen Peptidrest bestehend aus 1 - 30 Aminosäuren, wobei es sich um modifizierte und unmodifizierte D-Aminosäuren, L-Aminosäuren sowie unnatürliche Aminosäuren handeln kann, bedeuten,
   wobei mindestens einer der Reste R1-R10 eine Gruppe -(Y)ₙ-COOM, -(Y)ₙ-COOR13 oder - (Y)ₙ-C(O)NR14R15 ist,
   wobei Y -CH₂-, -CH(CH₃)-, -CH₂-CH₂-, -(CH₂)₃-, -CH=CH-, -C≡C-, -O-CH₂-CH₂-, -O-CH₂-CH₂-O-CH₂-CH₂- oder -NH-CH₂-CH₂- darstellt und n = 0 oder 1 ist,
   R11 C1-C10 bedeutet, und
   R12, R13 unabhängig voneinander Wasserstoff oder einen C1-C10-Alkylrest bedeuten.
2. Verbindung gemäß Punkt 1, wobei in der allgemeinen Formel 1
   der Rest R11 eine Ethylgruppe ist,
   die Reste R9, R10 Methylgruppen sind,
   die Reste R1, R4, R5, R6, R7, R8 Wasserstoff bedeuten,
   einer der Reste R2 und R3 Wasserstoff und der andere -(Y)ₙ-COOR13, -(Y)ₙ-COOM mit M = Na, K, [N(R12)₄]⁺ oder -(Y)ₙ-C(O)NR14R15 bedeutet
   oder die Reste R2 und R3 unabhängig voneinander -(Y)ₙ-COOR13, -(Y)ₙ-COOM mit M = Na, K, [N(R12)₄]⁺ oder -(Y)ₙ-C(O)NR14R15 bedeuten,
   wobei die Reste R14 und R15 unabhängig voneinander Wasserstoff, einen C1-C12-Alkyl-oder einen Peptidrest bestehend aus 1 - 30 Aminosäuren (wobei es sich um modifizierte und unmodifizierte D-Aminosäuren, L-Aminosäuren sowie unnatürliche Aminosäuren handeln kann) bedeuten,
   Y einen organischen Rest darstellt, wobei n = 0 oder 1 ist, und
   die Reste R12, R13 unabhängig voneinander Wasserstoff oder einen C1-C10-Alkylrest bedeuten.
3. Substrat, umfassend eine Verbindung gemäß Punkt 1 oder 2, **dadurch gekennzeichnet,** dass die Verbindung über einen Peptidrest oder einen Kohlenwasserstoffrest der Verbindung, optional über eine zusätzliche Linkergruppe, insbesondere eine Peptid-, Polyester-, Polyamid, Kohlenwasserstoff- oder Polyethylenglycol-Linkergruppe, an eine Oberfläche aus Metall, Keramik, Glas oder Polymermaterial, wobei es sich bei dem Polymermaterial vorzugsweise um ein Harz handelt, gebunden ist.
4. Verfahren zur Herstellung einer Verbindung gemäß Punkt 1 oder 2, **dadurch gekennzeichnet, dass** die Ausgangsverbindungen der Formel 2 und der Formel 3 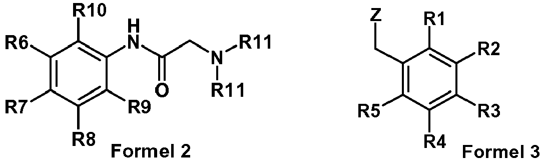 in fester oder gelöster Form miteinander umgesetzt werden, wobei Z in Formel 3 eine substituierbare Gruppe, insbesondere ein Halogenatom oder eine Tosylatgruppe, bedeutet, und die Reste R11 und R1-R10 die in Punkt 1 oder 2 definierte Bedeutung haben.
5. Verfahren gemäß Punkt 4, **dadurch gekennzeichnet, dass** das molare Verhältnis der Ausgangsverbindungen der Formel 2 und der Formel 3 1:1 beträgt und/oder die Umsetzung bei einer Temperatur von 20 - 200 °C, vorzugsweise bei 20 - 80 °C, erfolgt und die Umsetzung mit oder ohne Lösungsmittel erfolgt, wobei es sich bei dem optionalen Lösungsmittel um ein organisches Lösungsmittel oder Wasser handelt.
6. Verfahren gemäß Punkt 4 oder 5, dadurch gekennzeichnet, dass die beiden Ausgangsverbindungen der Formel 2 und der Formel 3 in fester Form bei Raumtemperatur (20 bis 30 °C) miteinander vermischt werden und ohne Lösungsmittel umgesetzt werden.
7. Verfahren gemäß Punkt 4 oder 5, **dadurch gekennzeichnet, dass** die Ausgangsverbindungen der Formel 2 und der Formel 3 in einer Mikrowellen-assistierten Reaktion innerhalb von 1 h miteinander umgesetzt werden, wobei die Mikrowellen-assistierte Reaktion in einem Lösungsmittel durchgeführt wird.
8. Verfahren zur Herstellung eines Substrats gemäß Punkt 3, **dadurch gekennzeichnet, dass** die Ausgangsverbindungen der Formel 2 und der Formel 3 wie in Punkt 4 definiert in einem Lösungsmittel miteinander umgesetzt werden, wobei eine der Ausgangsverbindungen der Formel 2 und der Formel 3 Festphasen-gebunden ist und die andere Ausgangsverbindung der Formel 2 und der Formel 3 in Lösung vorliegt, wobei die Festphasen-Bindung über einen der Reste R1-R10, optional über eine zusätzliche Linkergruppe, insbesondere eine Peptid-, Polyester-, Polyamid, Kohlenwasserstoff- oder Polyethylenglycol-Linkergruppe, erreicht wird, und es sich bei der festen Phase um Metall, Keramik, Glas oder ein Polymermaterial handelt, wobei es sich bei dem Polymermaterial vorzugsweise um ein Harz handelt.
9. Verwendung einer Verbindung gemäß einem der Punkte 1-2 als Geschmacksstoff, insbesondere als Bitterstoff.
10. Verwendung gemäß Punkt 9 in der Medizin, Biologie, Medizintechnik sowie in der Kosmetik, der pharmazeutischen, chemischen und Nahrungsmittelindustrie.

### Darstellung der Erfindung

Der Erfindung liegt daher die Aufgabe zugrunde, auf einfache Art und Weise Bitterstoffe herzustellen, die mindestens eine funktionelle Gruppe enthalten, bei deren Derivatisierung der bittere Geschmack erhalten bleibt oder durch geeignete hydrolytische bzw. enzymatische Spaltung der bittere Geschmack eintritt.

Erfindungsgemäß wird die Aufgabe gelöst, indem 2-Diethylamino-N-(2,6-dimethylphenyl)acetamid (Lidocain) oder ein funktionalisiertes Lidocainderivat mit Benzylhalogenidderivaten in geeigneter Weise zu Verbindungen der allgemeinen Formel 1 umgesetzt werden.

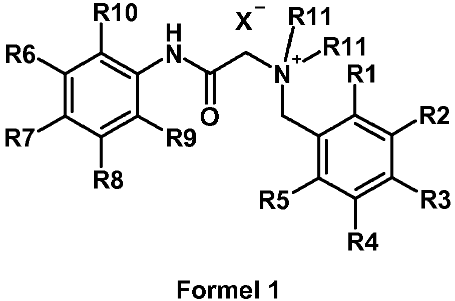

Dabei bedeutet X⁻ in Formel 1 beispielsweise Halogenid, Pseudohalogenid, Sulfat, Benzoat, Acetat, Trifluoracetat, Hydroxid, Saccharinat oder Capsaicinat. Bevorzugt ist X⁻ ein Halogenid und besonders bevorzugt Cl⁻, Br oder I⁻.

In Formel 1 bedeuten R1-R10 = H, Halogen, eine C1-C5-Alkyl-, C1-C4-Alkoxy-, C1-C20-Alkoxycarbonyl-, NH-P, O-P wobei P ein Wasserstoff, ein Peptidrest bzw. zur Kupplung modifizierter Peptidrest bestehend aus 1 - 30 Aminosäuren (modifizierte und unmodifizierte D-Aminosäuren, L-Aminosäuren sowie unnatürliche Aminosäuren wie definiert im "World Intellectual Property Organization (WIPO) Handbook on Industrial Property Information and Documentation, Standard ST.25 (1998), including Tables 1 through 6 of Appendix 2"), -(Y)ₙ-COOR13-, -(Y)ₙ-COOM- mit M = Na, K, [N(R12)₄]⁺ oder -(Y)ₙ-C(O)NR14R15-Gruppe, wobei R14 und R15 = H, einen C1-C12-Alkyl- oder einen Peptidrest bestehend aus 1 - 30 Aminosäuren (WIPO) bedeuten, wobei mindestens einer der Reste R1-R10 eine Gruppe - (Y)ₙ-COOM, -(Y)ₙ-COOR13 oder -(Y)ₙ-C(O)NR14R15 ist.

Y stellt einen organischen Rest, der aus der Gruppe -CH₂-, -CH(CH₃)-, -CH₂-CH₂-, -(CH₂)₃-, -CH=CH-, -C≡C-, -O-CH₂-CH₂-, -O-CH₂-CH₂-O-CH₂-CH₂-, und -NH-CH₂-CH₂- gewählt wird, wobei n = 0 oder 1 ist.

R11, R12, R13 bedeuten unabhängig voneinander H oder einen C1-C10-Alkylrest.

Bevorzugt sind R1-R5, R6-R10 = H, eine Methyl-, Ethyl-, Methoxy-, Ethoxy-, Carboxyl-, Methoxycarbonyl-, Ethoxycarbonyl- oder -(Y)ₙ-C(O)NR14R15-Gruppe mit R14 = H und R15 = C1-C12-Alkyl- oder einen Peptidrest bestehend aus 1 - 30 Aminosäuren (gemäß vorhergehender Definition), wobei einer der Reste R1-R5, R6-R10 eine -(Y)ₙ-COOR13-, -(Y)ₙ-COOM- oder -(Y)ₙ-C(O)NR14R15-Gruppe darstellt.

Besonders bevorzugt sind R1-R5 = H und einer der Reste R1-R3 eine Gruppe -(Y)ₙ-COOR13-, -(Y)ₙ-COOM- oder -(Y)ₙ-C(O)NR14R15.

Besonders bevorzugt sind R6-R8 = H und R9-R10 = Methyl, wenn mindestens einer der Reste R1-R5 eine Gruppe -(Y)ₙ-COOM, -(Y)ₙ-COOR13 oder -(Y)ₙ-C(O)NR14R15 ist.

Bevorzugt ist R11 ein C1-C8-Alkylrest wie z. B. eine Methyl-, Ethyl-, n-Propyl-, i-Propyl-, n-Butyl-Gruppe.

Besonders bevorzugt ist R11 eine Methyl-, Ethyl- oder Propylgruppe.

Bereits bekannt ist die Synthese von Denatoniumsalzen ausgehend von Lidocain und Benzylbromid in geeigneten Lösungsmitteln wie Alkohole, Wasser in einer mehrstündigen Umsetzung von 20 - 24 h bei erhöhter Temperatur zwischen 50 - 100 °C [WO2006062406 A2]. Für die Herstellung derartiger Verbindungen wären auch eine Amidkupplung aus entsprechenden Edukten Ph-NH₂ und Ph-CH₂-NR₂-CH₂COX1 (X1 = Halogen, OH) bzw. Ammoniumbildung ausgehend von Ph-NH-CO-CH₂-Hal (Hal = Halogen) und Ph-CH₂-NR₂ denkbar, was aber in beiden Varianten nur über lange Synthesewege und geringe Gesamtausbeute möglich ist.

Überraschend wurde gefunden, dass carboxylierte und carboxyalkyl-substituierte (-Alkyl-COOR) Benzylhalogenide mit Lidocain im molaren Verhältnis von 1:1 schon bei niedrigen Temperaturen bei 20 - 80 °C in einem geeigneten Lösungsmittel zu entsprechenden Carbonsäurederivaten des Denatonium reagieren. Auch ist eine Mikrowellen-assistierte Reaktion in einem geeigneten Lösungsmittel zur Herstellung von Substanzen gemäß Formel 1 bei Temperaturen bis 200 °C und Reaktionszeiten von max. 60 min für die Synthese geeignet. Noch überraschender war der Befund, dass die festen Substanzen auch ohne Verwendung von Lösungsmitteln nach Zusammengabe bei Raumtemperatur miteinander reagieren und dass die spontane Reaktion bei kurzen Reaktionszeiten von nur wenigen Minuten in hoher Ausbeute beendet ist. Auf diese Weise erhält man carboxylfunktionalisierte Denatoniumderivate, die sich weiter mittels geeigneter Synthese zu Salzen, Estern und Amiden umsetzen lassen.

Zudem wurde auch überraschend gefunden, dass die carboxylfunktionalisierten Denatoniumderivate einen intensiven bitteren Geschmack aufweisen, wohingegen Änderungen der Denatoniumgrundstruktur zum Verlust des bitteren Geschmacks führen. Vorteilhaft für die Synthese der carboxylfunktionalisierten Denatoniumderivate ist der geringe technische und energetische Aufwand, da entweder keine Lösungsmittel benötigt werden und in der Regel eine Reaktionsführung bei Raumtemperatur ausreichend ist oder nur kurze Reaktionszeiten in der Mikrowellen-assistierten Umsetzung nötig sind. Die Produkte zeichnen sich durch eine hohe Reinheit aus, wodurch eine aufwendige Reinigung entfällt.

Die unkomplizierte Herstellung der carboxylierten Denatoniumderivate ermöglicht die Bereitstellung derartiger Substanzen in größeren Mengen.

### Wege zur Ausführung der Erfindung

In einer bevorzugten Ausführungsform der Erfindung wird eines der beiden Edukte (Lidocain- oder Benzylhalogenidderivat) im Reaktionsgefäß fest vorgelegt. Bei Raumtemperatur wird die zweite Komponente fest zugegeben. Nach kurzem Mischen setzt eine spontane Reaktion ein, wobei die festen Substanzen eine Schmelze bilden. Mittels Dünnschichtchromatographie erfolgt die Reaktionskontrolle. In den meisten Fällen ist die Reaktion in 10 - 120 min abgeschlossen. Die Umsetzung kann ebenso in einem geeigneten Lösungsmittel wie einem organischen Lösungsmittel oder Wasser bei Raumtemperatur oder kurzzeitiger mäßiger Erwärmung erfolgen. Eine weitere Möglichkeit stellt die Mikrowellen-assistierte Umsetzung bis 200 °C bei Reaktionszeiten von max. 1 h dar. Eine Reinigung der Substanzen kann, falls nötig, mittels Umkristallisation oder Säulenchromatographie erfolgen. Für die weitere Derivatisierung werden geeignete Denatoniumderivate nach den dem Fachmann bekannten Methoden zu Salzen, Estern und Amiden umgesetzt.

Die Testung des bitteren Geschmacks synthetisierter Verbindungen im Vergleich zum Denatoniumbenzoat kann beispielsweise mit einer elektronischen Zunge durchgeführt werden, wobei nach dem Fachmann bekannten Methoden die elektrischen Potentiale wässriger Substanzlösungen definierter Konzentration bestimmt werden.

Wie Aminosäuren können Denatoniumderivate mittels Festphasenpeptidsynthese (SPPS) an eine immobilisierte (an ein polymeres Trägermaterial (Harz) gebundene) beliebig lange Aminosäurenkette gekuppelt werden. Bei der Synthese wird die sogenannte Fmoc-Chemie mit HOBt (1-Hydroxybenzotriazol) und DIPEA (*N*,*N*-Diisopropylethylamin) als Hilfsbase angewendet. HOBt erzeugt aus der Carboxylgruppe der zu kuppelnden Fmoc-Aminosäure zunächst einen Aktivester, der dann mit Aminen zu einer Peptidbindung reagiert. Die Fmoc-Abspaltung erfolgt mit Piperidin in Dimethylformamid (DMF).

Nach erfolgter Kupplung erfolgt simultan die Abspaltung der Schutzgruppen, sowie des aus Denationumderivat und Peptid bestehenden Konstrukts unter Zusatz von TFA vom Trägermaterial. Bei der Abspaltung entstehende reaktive Carbokationen werden durch die Scavenger Ethandithiol, m-Kresol und Thioanisol gequencht.

Anschließend wird das vom Trägermaterial abgespaltene Denatoniumderivat/Peptid-Konstrukt in eiskaltem Ether gefällt und nach Zentrifugation die Lösung vom Konstrukt-Pellet entfernt.

Eine erfolgte Kupplung kann mittels Massenspektrometrie, (Elektrospray-Ionisation (ESI) bzw. Matrix-unterstützte Laser-Desorption/Ionisation (MALDI)) überprüft werden.

Die nachstehenden Beispiele dienen der näheren Erläuterung der Erfindung, ohne diese einzuschränken.

### Beispiel 1

### Umsetzung von Lidocain mit 4-(Brommethyl)benzoesäuremethylester

17 mg Lidocain (72,5 µmol) werden mit einem geringen Überschuss an 4-(Brommethyl)benzoesäuremethylester (18 mg, 80 µmol) versetzt und in 3 ml Wasser suspendiert. Die Suspension wird für 30 min bei 200 °C in der Mikrowelle belassen. Die Lösung wird anschließend eingeengt und in 1 ml THF aufgenommen. Der entstandene weiße Feststoff wird abfiltriert und mit THF gewaschen. Man erhält 2-(2,6-Dimethylphenylamino)-N,N-diethyl-N-(4-(methoxycarbonyl)benzyl)-2-oxoethanammoniumbromid.

### Beispiel 2

### Umsetzung von Lidocain mit 4-(Brommethyl)benzoesäure

1 g Lidocain (4,3 mmol) werden mit 0,92 g (4,3 mmol) 4-(Brommethyl)benzoesäure unter Schütteln gemischt. Zur Vervollständigung des Umsatzes wird 20 Minuten auf 50 °C erwärmt. Anschließend werden 10 ml THF zugegeben und gerührt. Der entstandene weiße Niederschlag wird abfiltriert, mit THF gewaschen und getrocknet. Die Substanz wird vom entstandenen Nebenprodukt Lidocain-4-(bromomethyl)benzoat säulenchromatographisch abgetrennt und man erhält N-(4-Carboxybenzyl)-2-(2,6-dimethylphenylamino)-N,N-diethyl-2-oxoethanammoniumbromid.

### Beispiel 3

### Synthese von 2-(2,6-Dimethylphenylamino)-N,N-diethyl-N-(3-(methoxycarbonyl)benzyl)-2-oxoethanammoniumbromid

1 g Lidocain (4,3 mmol) werden mit 0,98 g (4,3 mmol) 3-(Brommethyl)-benzoesäuremethylester zusammengegeben und bei Raumtemperatur innerhalb von 10 Minuten nach der Zugabe mehrfach geschüttelt. Anschließend werden 10 ml THF zugegeben und gerührt. Der entstandene weiße Niederschlag wird abfiltriert, mit THF gewaschen und getrocknet. Man erhält 2-(2,6-Dimethylphenylamino)-N,N-diethyl-N-(3-(methoxycarbonyl)benzyl)-2-oxoethanammoniumbromid.
m.p.: 183 - 184 °C
MS (ESI): m/z = 383,233 Da (C₂₃H₃₁N₂O₃)⁺
¹H-NMR (DMF-D7, ppm): 1,61-1,64 (t, 6H, 2 x CH₃); 2,32 (s, 6H, 2 x CH₃); 2,73-2,78; 2,91-2,95 (CH₃, DMF-D7); 3,52 (H₂O in DMF); 3,71-3,81 (m, 4H, 2 x CH₂); 3,94 (s, 3H, O-CH₃); 4,68 (s, 2H, CH₂); 5,20 (s, 2H, CH₂); 7,13-7,16 (m, 3H, 3 x CH); 7,72-7,75 (t, 1H, CH); 8,03 (CH, DMF); 8,08-8,10 (d, 1H, CH); 8,16-8,18 (d, 1H,CH); 8,38 (s, 1H, CH); 10,87 (s, 1H, NH) ¹³C-NMR (DMF-D7, ppm): 8,54 (CH₃); 18,77 (CH₃); 29,59-30,60 (DMF-D7); 34,73-35,73 (DMF-D7); 52,69 (O-CH₃); 55,39 (CH₂); 56,66 (CH₂); 62,11 (CH₂); 127,80 (CH); 128,60 (CH); 129,46 (C); 130,26 (CH); 131,52 (C); 131,75 (CH); 134,55 (CH); 134,62 (C); 136,08 (C); 138,56 (CH); 162,60-162,92 (DMF-D7); 163,45 (C); 166,55 (C)

### Beispiel 4

### Synthese von 2-(2,6-Dimethylphenylamino)-N,N-diethyl-N-(4-(methoxycarbonyl)benzyl)-2-oxoethanammoniumbromid

1 g Lidocain (4,3 mmol) werden mit 0,98 g (4,3 mmol) 4-(Brommethyl)benzoesäure-methylester zusammengegeben und bei Raumtemperatur innerhalb von 20 Minuten nach der Zugabe mehrfach geschüttelt. Anschließend werden 10 ml THF zugegeben und gerührt. Der entstandene weiße Niederschlag wird abfiltriert, mit THF gewaschen und getrocknet. Man erhält 2-(2,6-Dimethylphenylamino)-N,N-diethyl-N-(4-(methoxycarbonyl)benzyl)-2-oxoethanammoniumbromid.
m.p.: 176 - 178 °C
¹H-NMR (DMF-D7, ppm): 1,61-1,64 (t, 6H, 2 x CH₃); 2,31 (s, 6H, 2 x CH₃); 2,73-2,78; 2,90-2,95 (CH₃, DMF-D7); 3,53 (H₂O in DMF); 3,74-3,82 (m, 4H, 2 x CH₂); 3,96 (s, 3H, O-CH₃); 4,72 (s, 2H, CH₂); 5,16 (s, 2H, CH₂); 7,12-7,17 (m, 3H, 3 x CH); 7,97-7,98 (d, 2H, 2 x CH); 8,03 (CH, DMF); 8,10-8,11 (d, 2H, 2 x CH); 10,88 (s, 1H, NH)
¹³C-NMR (DMF-D7, ppm): 8,83 (CH₃); 18,95 (CH₃); 29,76-30,76 (DMF-D7); 34,89-35,89 (DMF-D7); 52,89 (O-CH₃); 55,90 (CH₂); 57,18 (CH₂); 62,22 (CH₂); 127,94 (CH); 128,74 (CH); 130,48 (CH); 132,40 (C); 133,80 (C); 134,51 (CH); 134,75 (C); 136,23 (C); 162,62-163,09 (DMF-D7); 163,55 (C); 166,73 (C)

### Beispiel 5

### Synthese von 2-(2,6-Dimethylphenylamino)-N,N-diethyl-N-(4-(methoxycarbonyl)benzyl)-2-oxoethanammoniumchlorid

1 g Lidocain (4,3 mmol) werden mit 0,79 g (4,3 mmol) 4-(Chlormethyl)-benzoesäuremethylester zusammengegeben und bei Raumtemperatur innerhalb von 20 Minuten nach der Zugabe mehrfach geschüttelt. Anschließend werden 10 ml THF zugegeben und gerührt. Der entstandene weiße Niederschlag wird abfiltriert, mit THF gewaschen und getrocknet. Man erhält 2-(2,6-Dimethylphenylamino)-N,N-diethyl-N-(4-(methoxycarbonyl)benzyl)-2-oxoethanammoniumchlorid.

### Beispiel 6

### Synthese von 2-(2,6-Dimethylphenylamino)-N,N-diethyl-N-(4-(2-methoxy-2-oxoethyl)benzyl)-2-oxoethanammoniumbromid

0,3 g Lidocain (1,3 mmol) werden mit 0,31 g (1,3 mmol) Methyl-2-(4-(bromomethyl)phenyl)acetat zusammengegeben und bei Raumtemperatur innerhalb von 20 Minuten nach der Zugabe mehrfach geschüttelt. Anschließend werden 10 ml THF zugegeben und gerührt. Der entstandene weiße Niederschlag wird abfiltriert, mit THF gewaschen und getrocknet. Man erhält 2-(2,6-Dimethylphenylamino)-N,N-diethyl-N-(4-(2-methoxy-2-oxoethyl)benzyl)-2-oxoethanammoniumbromid.
¹H-NMR (DMF-D7, ppm): 1,59 (m, 6H, 2 x CH₃); 2,32 (s, 6H, 2 x CH₃); 2,73; 2,91 (CH₃, DMF-D7); 3,63-3,69; (m, 6H, 3 x CH₂); 3,84 (s, 3H, O-CH₃); 4,70 (s, 2H, CH₂); 5,06 (s, 2H, CH₂); 7,14 (m, 3H, 3 x CH); 7,49-7,50 (d, 2H, 2 x CH); 7,76-7,77 (d, 2H, 2 x CH); 8,05 (CH, DMF); 10,99 (s, 1H, NH)
¹³C-NMR (DMF-D7, ppm): 8,82 (CH₃); 18,95 (CH₃); 29,70-30,71 (DMF-D7); 34,86-35,87 (DMF-D7); 40,54 (CH₂); 52,26 (O-CH₃); 55,43 (CH₂); 56,87 (CH₂); 62,52 (CH₂); 127,24 (C); 127,83 (CH); 128,63 (CH); 130,84 (CH); 133,91 (CH); 134,65 (C); 136,09 (C); 137,89 (C); 162,53-163,0 (DMF-D7); 163,41 (C); 172,09 (C)

### Beispiel 7

### Synthese von 2-(2,6-Dimethylphenylamino)-N,N-diethyl-N-(4-(1-methoxy-1-oxopropan-2-yl)benzyl)-2-oxoethanaminiumbromid

0,4 g Lidocain (1,7 mmol) werden mit 0,44 g (1,7 mmol) Methyl-2-(4-(bromomethyl)phenyl)propanoat zusammengegeben und mehrfach geschüttelt. Dabei wird zur Vervollständigung der Reaktion 20 Minuten auf 80 °C erwärmt. Anschließend werden 10 ml THF zugegeben und gerührt. Der entstandene weiße Niederschlag wird abfiltriert, mit THF gewaschen und getrocknet. Man erhält 2-(2,6-Dimethylphenylamino)-N,N-diethyl-N-(4-(1-methoxy-1-oxopropan-2-yl)benzyl)-2-oxoethanamin iumbromid.

### Beispiel 8

### Synthese von 2-(2,6-Dimethylphenylamino)-N-(4-(2-ethoxy-2-oxoethylcarbamoyl)benzyl)-N,N-diethyl-2-oxoethanammoniumbromid

0,5 g Ethyl-2-(4-(bromomethyl)benzamido)acetat (1,7 mmol) werden mit 0,39 g Lidocain (1,7 mmol) versetzt und bei 80 °C innerhalb von 20 Minuten nach der Zugabe mehrfach geschüttelt. Anschließend werden 10 ml THF zugegeben und gerührt. Der entstandene weiße Niederschlag wird abfiltriert, mit THF gewaschen und getrocknet. Man erhält 2-(2,6-Dimethylphenylamino)-N-(4-(2-ethoxy-2-oxoethylcarbamoyl)benzyl)-N,N-diethyl-2-oxoethanammoniumbromid.
¹H-NMR (D₂O, ppm): 1,31-1,34 (t, 3H, CH₃); 1,53-1,56 (t, 6H, 2 x CH₃); 2,25 (s, 6H, 2 x CH₃); 3,58-3,67; (m, 4H, 2 x CH₂); 4,22 (s, 4H, 2 x CH₂); 4,27-4,31; (q, 2H, CH₂); 4,89 (s, 2H, CH₂); 7,20-7,28 (m, 3H, 3 x CH); 7,68-7,69 (d, 2H, 2 x CH); 7,94-7,96 (d, 2H, 2 x CH);
¹³C-NMR (D₂O, ppm): 8,40 (CH₃); 14,38 (CH₃); 18,42 (CH₃); 42,94 (CH₂); 55,69 (CH₂); 56,35 (CH₂); 62,31 (CH₂); 63,59 (CH₂); 129,08(CH); 129,37 (CH); 129,49 (CH); 131,86 (C); 132,98 (C); 134,11 (CH); 136,02 (C); 136,74 (C); 164,70 (C); 170,73 (C); 172,60 (C)

### Beispiel 9

### Synthese von 2-(2,6-Dimethylphenylamino)-N-(4-(2-(2-ethoxy-2-oxoethylamino)-2-oxoethyl-carbamoyl)benzyl)-N,N-diethyl-2-oxoethanammoniumbromid

0,23 g Ethyl-2-(2-(4-(bromomethyl)benzamido)acetamido)acetat (0,6 mmol) werden mit 0,15 g Lidocain (0,6 mmol) zusammengegeben und bei 80 °C innerhalb von 20 Minuten nach der Zugabe mehrfach geschüttelt. Anschließend werden 10 ml THF zugegeben und gerührt. Der entstandene weiße Niederschlag wird abfiltriert, mit THF gewaschen und getrocknet. Man erhält 2-(2,6-Dimethylphenylamino)-N-(4-(2-(2-ethoxy-2-oxoethylamino)-2-oxoethyl-carbamoyl)benzyl)-N,N-diethyl-2-oxoethanammoniumbromid.

### Beispiel 10

### Synthese von N-(3-Carboxybenzyl)-2-(2,6-dimethylphenylamino)-N,N-diethyl-2-oxoethanammoniumbromid durch Verseifung mit NaOH

0,5 g 2-(2,6-Dimethylphenylamino)-N,N-diethyl-N-(3-(methoxycarbonyl)benzyl)-2-oxoethanammoniumbromid (1,08 mmol) werden mit einem Überschuss an NaOH (3,2 mmol, 129,4 mg) in Ethanol versetzt und drei Stunden zum Sieden erhitzt. Anschließend werden 25 ml 0,1 N HCl zugegeben. Die Lösung wird zur Trockne eingeengt und das ölige Produkt mit THF versetzt und gerührt. Der entstandene weiße Niederschlag wird abfiltriert und mit THF gewaschen. Man erhält N-(3-Carboxybenzyl)-2-(2,6-dimethylphenylamino)-N,N-diethyl-2-oxoethanammoniumbromid.
m.p.: 142 - 144 °C
MS: (ESI) m/z = 369,217 Da (C₂₂H₂₉N₂O₃)⁺
¹H-NMR (D₂O + NaOD, ppm): 1,50-1,55 (t, 6H, 2 x CH₃); 2,13 (s, 6H, 2 x CH₃); 3,44-3,60 (m, 4H, 2 x CH₂); 4,89 (s, 2H, CH₂ + D₂O); 4,91 (s, 2H, CH₂); 6,94-6,97 (t, 1H, CH); 7,09-7,11 (d, 2H, 2 x CH); 7,59-7,62 (t, 1H, CH); 7,80-7,82 (d, 1H,CH); 8,00-8,01 (d, 1H, CH); 8,06 (s, 1H, CH)
¹³C-NMR (D₂O + NaOD, ppm): 10,41 (CH₃); 20,56 (CH₃); 56,35 (CH₂); 63,53 (CH₂); 70,66 (CH₂); 125,54 (CH); 130,32 (CH); 130,51 (C); 131,92 (CH); 133,47 (CH); 133,88 (C); 135,68 (CH); 138,17 (CH); 140,00 (C); 149,15 (C); 163,75 (C); 177,55 (C)

### Beispiel 11

### Synthese von N-(3-Carboxybenzyl)-2-(2,6-dimethylphenylamino)-N,N-diethyl-2-oxoethanammoniumbromid durch Verseifung mit Ba(OH)₂

0,5 g 2-(2,6-Dimethylphenylamino)-N,N-diethyl-N-(3-(methoxycarbonyl)benzyl)-2-oxoethanammoniumbromid (1,08 mmol) werden mit 30 ml einer 0,05 M Ba(OH)₂-Lösung in Ethanol versetzt und drei Stunden zum Sieden erhitzt. Anschließend werden 30 ml einer 0,05 M H₂SO₄-Lösung zugegeben. Der Niederschlag hauptsächlich bestehend aus BaSO₄ wird abfiltriert. Anschließend wird die Lösung zur Trockne eingeengt und das kristalline Produkt mit THF gewaschen. Man erhält N-(3-Carboxybenzyl)-2-(2,6-dimethylphenylamino)-N,N-diethyl-2-oxoethanammoniumsulfat.

### Beispiel 12

### Kupplung von N-(3-Carboxybenzyl)-2-(2,6-dimethylphenylamino)-N,N-diethyl-2-oxoethanammoniumbromid mit dem Peptid (Fmoc)G-P-Q-G-I-A-G-Q-A(N3)-Q-Harz

Das Peptid (Fmoc)G-P-Q-G-I-A-G-Q-A(N3)-Q-Harz wird mittels üblicher Peptidsynthese (Fmoc-Strategie) schrittweise an einem Syntheseharz aufgebaut. Zur Anbindung des Bitterstoffes wird die Fmoc-Schutzgruppe der N-terminalen endständigen Aminosäure Glycin mittels 40 %- und 20 %-Piperidinlösung in DMF abgespalten. 83,5 µmol des Denatoniumderivates werden in 500 µl einer 0,5 M HOBt Lösung aus H₂O/DMF 1:4 gelöst. Das Harz wird mit der Lösung und anschließend mit 20 µl Diisopropylcarbodiimid (DIC) versetzt und eine Stunde bei Raumtemperatur geschüttelt. Anschließend wird das Harz mehrfach mit DMF, Dichlormethan und Diethylether gewaschen. Man erhält ein Harz-gebundenes Peptid-Bitterstoff-Kupplungsprodukt.

Nach durchgeführter Kupplung erfolgt simultan die Abspaltung der Schutzgruppen, sowie des aus Denatoniumderivat und Peptid bestehenden Produkts unter Zusatz von TFA vom Trägermaterial. Bei der Abspaltung entstehende reaktive Carbokationen werden durch die Scavenger Ethandithiol, m-Kresol und Thioanisol gequencht.

Anschließend wird das vom Trägermaterial abgespaltene Denatoniumderivat/Peptid Konstrukt in eiskaltem Ether gefällt und nach Zentrifugation die Lösung vom entstandenen Pellet entfernt. Eine erfolgte Kupplung kann mittels Massenspektrometrie (Elektrospraylonisation (ESI) bzw. Matrix-unterstützte Laser-Desorption/Ionisation (MALDI)) überprüft werden.

### Beispiel 13

### Kupplung von N-(3-Carboxybenzyl)-2-(2,6-dimethylphenylamino)-N,N-diethyl-2-oxoethanammoniumbromid mit dem Peptid (Fmoc)G-P-Q-G-I-A-G-Q-A(N3)-Q-Harz

Das Peptid (Fmoc)G-P-Q-G-I-A-G-Q-A(N3)-Q-Harz wird mittels üblicher Peptidsynthese (Fmoc-Strategie) schrittweise an einem Syntheseharz aufgebaut. Zur Anbindung des Bitterstoffes wird die Fmoc-Schutzgruppe der endständigen Aminosäure Glycin mittels 40 %er und 20 %er Piperidinlösung in DMF abgespalten. 83,5 µmol des Denatoniumderivates werden in 500 µl einer 0,5 M HOBt Lösung aus H₂O/DMF 1:4 gelöst. Das Harz wird mit der Lösung und anschließend mit einer Lösung von 125 µmol *N*-Ethyl-*N'*-(3-dimethylaminopropyl)carbodiimid-hydrochlorid (EDC) in 100 µl H₂O/DMF 1:4 versetzt und eine Stunde bei Raumtemperatur geschüttelt. Anschließend wird das Harz mehrfach mit DMF, Dichlormethan und Diethylether gewaschen. Man erhält ein Harz-gebundenes Peptid-Bitterstoff-Kupplungsprodukt.

Nach durchgeführter Kupplung erfolgt simultan die Abspaltung der Schutzgruppen, sowie des aus Denatoniumderivat und Peptid bestehenden Produkts unter Zusatz von TFA vom Trägermaterial. Bei der Abspaltung entstehende reaktive Carbokationen werden durch die Scavenger Ethandithiol und Thioanisol gequencht.

Nach einer Inkubationszeit von 1,5 Stunden wird das vom Trägermaterial abgespaltene Denatoniumderivat/Peptid Konstrukt in eiskaltem Ether gefällt und nach Zentrifugation die Lösung vom entstandenen Pellet entfernt. Eine erfolgte Kupplung kann mittels Massenspektrometrie (Elektrospray-Ionisation (ESI) bzw. Matrix-unterstützte Laser-Desorption/Ionisation (MALDI)) überprüft werden.

### Beispiel 14

### Kupplung von N-(3-Carboxybenzyl)-2-(2,6-dimethylphenylamino)-N,N-diethyl-2-oxoethanammoniumbromid mit dem Peptid (Fmoc)NH-PEG(3)-Lys(Boc)-G-P-Q-G-I-A-G-Q-PEG(3)-Q-Harz

Das Peptid (Fmoc)NH-PEG(3)-Lys(Boc)-G-P-Q-G-I-A-G-Q-PEG(3)-Q-Harz wird mittels üblicher Peptidsynthese (Fmoc-Strategie) schrittweise an einem Syntheseharz aufgebaut. Zur Anbindung des Bitterstoffes wird die Fmoc-Schutzgruppe der endständigen Aminosäure NH₂-PEG(3)-COOH mittels 40 %er und 20 %er Piperidinlösung in DMF abgespalten. 83,5 µmol des Denatoniumderivates werden in 500 µl einer 0,5 M HOBt Lösung aus H₂O/DMF 1:4 gelöst. Das Harz wird mit der Lösung und anschließend mit 20 µl Diisopropylcarbodiimid (DIC) versetzt und eine Stunde bei Raumtemperatur geschüttelt. Anschließend wird das Harz mehrfach mit DMF, Dichlormethan und Diethylether gewaschen. Man erhält ein Harz-gebundenes Peptid-Bitterstoff-Kupplungsprodukt.

Nach durchgeführter Kupplung erfolgt simultan die Abspaltung der Schutzgruppen, sowie des aus Denatoniumderivat und Peptid bestehenden Produkts unter Zusatz von TFA vom Trägermaterial. Bei der Abspaltung entstehende reaktive Carbokationen werden durch die Scavenger Ethandithiol, m-Kresol und Thioanisol gequencht.

Nach einer Inkubationszeit von 1,5 Stunden wird das vom Trägermaterial abgespaltene Denatoniumderivat/Peptid Konstrukt in eiskaltem Ether gefällt und nach Zentrifugation die Lösung vom entstandenen Pellet entfernt. Eine erfolgte Kupplung kann mittels Massenspektrometrie (Elektrospray-Ionisation (ESI) bzw. Matrix-unterstützte Laser-Desorption/Ionisation (MALDI) überprüft werden.
MS (MALDI-TOF): m/z = 1739

### Beispiel 15

### Kupplung von N-(3-Carboxybenzyl)-2-(2,6-dimethylphenylamino)-N,N-diethyl-2-oxoethanammoniumbromid mit dem Peptid (Fmoc)NH-PEG(3)-Lys(Boc)-G-P-Q-G-I-A-G-Q-PEG(3)-A(N3)-QHarz

Das Peptid (Fmoc)NH-PEG(3)-Lys(Boc)-G-P-Q-G-I-A-G-Q-PEG(3)-A(N3)-QHarz wird mittels üblicher Peptidsynthese (Fmoc-Strategie) schrittweise an einem Syntheseharz aufgebaut. Zur Anbindung des Bitterstoffes wird die Fmoc-Schutzgruppe der endständigen Aminosäure NH₂-PEG(3)-COOH mittels 40 % und 20 % Piperidinlösung in DMF abgespalten. 83,5 µmol des Denatoniumderivates werden in 500 µl einer 0,5 M HOBt Lösung aus H₂O/DMF 1:4 gelöst. Das Harz wird mit der Lösung und anschließend mit 20 µl Diisopropylcarbodiimid (DIC) versetzt und eine Stunde bei Raumtemperatur geschüttelt. Anschließend wird das Harz mehrfach mit DMF, Dichlormethan und Diethylether gewaschen. Man erhält ein Harz-gebundenes Peptid-Bitterstoff-Kupplungsprodukt.

Nach durchgeführter Kupplung erfolgt simultan die Abspaltung der Schutzgruppen, sowie des aus Denatoniumderivat und Peptid bestehenden Produkts unter Zusatz von TFA vom Trägermaterial. Bei der Abspaltung entstehende reaktive Carbokationen werden durch die Scavenger Ethandithiol, m-Kresol und Thioanisol gequencht.

Nach einer Inkubationszeit von 1,5 Stunden wird das vom Trägermaterial abgespaltene Denatoniumderivat/Peptid Konstrukt in eiskaltem Ether gefällt und nach Zentrifugation die Lösung vom entstandenen Pellet entfernt. Eine erfolgte Kupplung kann mittels Massenspektrometrie (Elektrospray-Ionisation (ESI) bzw. Matrix-unterstützte Laser-Desorption/Ionisation (MALDI) überprüft werden.
MS (MALDI-TOF): m/z = 1852

### Beispiel 16

### Enzymatische Peptidspaltung I

Die in den Beispielen 12-15 aufgeführten Peptide können nach Abspaltung von dem Harz durch Matrix Metallo-Proteasen (MMP) wie MMP-8 spezifisch gespalten werden. Dafür erfolgt eine 2-3 stündige Aktivierung der pro-MMP durch p-Aminophenyl-Quecksilberacetat (APMA 10 mM in 0,1 M NaOH) im Verhältnis 10:1 (MMP:APMA v/v) bei 37 °C. Die aktivierte MMP wird anschließend in definierten Konzentrationen (9 ng/ml, 45 ng/ml, 90 ng/ml, 225 ng/ml, 450 ng/ml sowie 900 ng/ml) zu den in Puffer (200 mM NaCl, 50 mM Tris-HCI, 5 mM CaCl₂, 1 µM ZnCl₂, 0.05 % BRIJ® 35, 0.05 % NaN₃, pH 7.0) gelösten Peptiden gegeben und 1 h bei 37 °C inkubiert. Die Reaktion wird durch Abtrennung/Filtration der MMP durch Zentrifugation oder Zugabe von 10 Äquivalenten EDTA (250 mM) gestoppt. Eine Analyse der Spaltprodukte erfolgt durch Flüssigchromatographie mit Massenspektrometrie-Kopplung (LC-MS).

### Beispiel 17

### Enzymatische Peptidspaltung II

Das durch den Austausch von L- zu D-Aminosäuren modifizierte Peptid NH2-G-p-q-G-I-AG-q-Q-COOH (Kleinbuchstaben stehen für D-Aminosäuren) wird nach Abspaltung von dem Harz durch MMPs wie MMP-8 spezifisch gespalten. Dafür erfolgt eine 2-3 stündige Aktivierung der pro-MMP durch p-Aminophenyl-Quecksilberacetat (APMA 10 mM in 0,1 M NaOH) im Verhältnis 10:1 (MMP:APMA v/v) bei 37 °C. Die aktivierte MMP wird anschließend in definierten Konzentrationen zu den in Puffer (200 mM NaCl, 50 mM Tris-HCI, 5 mM CaCl₂, 1 µM ZnCl₂, 0.05 % BRIJ® 35, 0.05 % NaN₃, pH 7.0) gelösten Peptiden gegeben und 1 h bei 37 °C inkubiert. Die Reaktion wird durch Abtrennung/Filtration der MMP durch Zentrifugation oder Zugabe von 10 Äquivalenten EDTA (250 mM) gestoppt. Eine Analyse der Spaltprodukte erfolgt durch Flüssigchromatographie mit Massenspektrometrie-Kopplung (LC-MS).

### Beispiel 18

### Geschmackstestung mittels elektronischer Zunge von 2-(2,6-Dimethylphenylamino)-N,N-diethyl-N-(3-(methoxycarbonyl)benzyl)-2-oxoethanammoniumbromid

Mittels elektronische Zunge werden die Potentiale wässriger Lösungen der Substanz mit Denatoniumbenzoat (in Klammern) verglichen und liegen im Bereich von etwa: Sensortyp SB2AC0: 0,05 mM = -75 mV (-75 mV); 0,1 mM = -65 mV (-61,5 mV); 0,5 mM = -32 mV (-30 mV); 1 mM = -20 mV (-24 mV); Sensortyp SB2AN0: 0,05 mM = -90 mV (-90 mV); 0,1 mM = -88,5 mV (-85 mV); 0,5 mM = -60 mV (-60 mV); 1 mM = -21,5 mV (-26 mV); Sensortyp SB2BT0: 0,05 mM = -73 mV (-72 mV); 0,1 mM = -72 mV (-54 mV); 0,5 mM = 7 mV (-2 mV); 1 mM = 44 mV (47 mV). Die gemessenen elektrischen Potentiale zeigen eine stark bittere Substanz an.

### Beispiel 19

### Geschmackstestung mittels elektronischer Zunge von 2-(2,6-Dimethylphenylamino)-N-(4-(2-ethoxy-2-oxoethylcarbamoyl)benzyl)-N,N-diethyl-2-oxoethanammoniumbromid

Mittels elektronische Zunge werden die Potentiale wässriger Lösungen der Substanz mit Denatoniumbenzoat (in Klammern) verglichen und liegen im Bereich von etwa: Sensortyp SB2AC0: 0,05 mM = -93 mV (-72 mV); 0,1 mM = -88 mV (-57 mV); 0,5 mM = -52 mV (-23 mV); 1 mM = -42 mV (-6 mV); Sensortyp SB2AN0: 0,05 mM = -70 mV (-60 mV); 0,1 mM = - 60 mV (-50 mV); 0,5 mM = -30 mV (-1 mV); 1 mM = -10 mV (20 mV); Sensortyp SB2BT0: 0,05 mM = -95 mV (-80 mV); 0,1 mM = -94 mV (-62 mV); 0,5 mM = -35 mV (7 mV); 1 mM = 5 mV (42 mV). Die gemessenen elektrischen Potentiale zeigen eine stark bittere Substanz an.

### Beispiel 20

### Synthese von 2-(2,6-Dimethylphenylamino)-N,N-diethyl-N-(3-methoxybenzyl)-2-oxoethanammoniumbromid

1 g Lidocain (4,3 mmol) werden mit 0,86 g (4,3 mmol) 3-Methoxybenzylbromid zusammengegeben und bei Raumtemperatur innerhalb von 20 Minuten nach der Zugabe mehrfach geschüttelt. Anschließend werden 10 ml THF zugegeben und gerührt. Der entstandene weiße Niederschlag wird abfiltriert, mit THF gewaschen und getrocknet. Man erhält 2-(2,6-Dimethylphenylamino)-N,N-diethyl-N-(3-methoxybenzyl)-2-oxoethanammoniumbromid.
m.p.: 144 - 151 °C
¹H-NMR (CDCl₃, ppm): 1,52-1,55 (t, 6H, 2 x CH₃); 2,27 (s, 6H, 2 x CH₃); 3,45-3,52 (qd, 2H, CH₂); 3,59-3,66 (qd, 2H, CH₂); 3,81 (s, 3H, CH₃); 4,65 (s, 2H, CH₂); 4,85 (s, 2H, CH₂); 7,02-7,11 (m, 6H, CH); 7,35-7,38 (m, 1H,CH); 10,48 (s, 1H, NH)

### Beispiel 21

### Synthese von 2-(2,6-Dimethylphenylamino)-N,N-diethyl-N-(3-hydroxybenzyl)-2-oxoethanammoniumbromid

0,5 g 2-(2,6-Dimethylphenylamino)-N,N-diethyl-N-(3-methoxybenzyl)-2-oxoethanammoniumbromid (1,1 mmol) werden in 10 ml Dichlormethan gelöst und gerührt. Bei -70 °C wird eine Lösung von Bortribromid (2,3 mmol) in 10 ml Methylenchlorid zugetropft. Bei erreichter Raumtemperatur wird das Gemisch mit 5 ml Wasser versetzt. Die organische Phase wird eingeengt und säulenchromatographisch über Aluminiumoxid gereinigt (Laufmittel: Methanol/Chloroform 1:9). Man erhält 2-(2,6-Dimethylphenylamino)-N,N-diethyl-N-(3-hydroxybenzyl)-2-oxoethanammoniumbromid.

### Beispiel 22

### Synthese von 2-(2,6-Dimethylphenylamino)-N,N-diethyl-N-(tert-butyl-4-(bromomethyl)-phenylcarbamat)-2-oxoethanammoniumbromid

0,5 g Lidocain (2,1 mmol) werden mit 0,61 g (2,1 mmol) tert-Butyl-4-(bromomethyl)phenylcarbamat zusammengegeben und mehrfach geschüttelt. Dabei wird zur Vervollständigung der Reaktion 20 Minuten auf 80 °C erwärmt. Anschließend werden 10 ml THF zugegeben und gerührt. Der entstandene weiße Niederschlag wird abfiltriert, mit THF gewaschen und getrocknet. Man erhält 2-(2,6-Dimethylphenylamino)-N,N-diethyl-N-(tert-butyl-4-(bromomethyl)-phenylcarbamat)-2-oxoethanammoniumbromid.

### Beispiel 23

### Synthese von 2-(2,6-Dimethylphenylamino)-N,N-diethyl-N-4-(aminobenzyl)-2-oxoethanammoniumbromid

0,5 g 2-(2,6-Dimethylphenylamino)-N,N-diethyl-N-(tert-butyl-4-(bromomethyl)-phenylcarb-amat)-2-oxoethanammoniumbromid (0,96 µmol) werden mit 1,9 µmol Trifluoressigsäure in 10 ml Methylenchlorid gerührt. Die organische Phase wird eingeengt und säulenchromatographisch über Aluminiumoxid gereinigt (Laufmittel: Methanol/Chloroform 1:9). Man erhält 2-(2,6-Dimethylphenylamino)-N,N-diethyl-N-4-(aminobenzyl)-2-oxoethanammoniumbromid.

## Patentansprüche

1. Verbindung der allgemeinen Formel 1 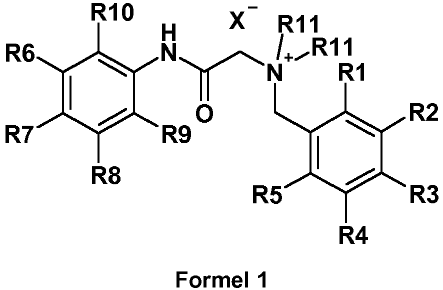 wobei
X⁻ Halogenid, Pseudohalogenid, Sulfat, Benzoat, Acetat, Trifluoracetat, Hydroxid, Saccharinat oder Capsaicinat bedeutet,
R1-R10 unabhängig voneinander Wasserstoff, Halogen, C1-C5-Alkyl-, C1-C4-Alkoxy-, C1-C20-Alkoxycarbonyl-, -NH-P, -O-P, wobei P ein Wasserstoff, ein Peptidrest bestehend aus 1 - 30 Aminosäuren, wobei es sich um modifizierte und unmodifizierte D-Aminosäuren, L-Aminosäuren sowie unnatürliche Aminosäuren handeln kann, ist, -(Y)ₙ-COOR13, -(Y)ₙ-COOM mit M = Na, K, [N(R12)₄]⁺ oder -(Y)ₙ-C(O)NR14R15 bedeuten,
wobei R14 und R15 Wasserstoff, einen C1-C12-Alkyl- oder einen Peptidrest bestehend aus 1 - 30 Aminosäuren, wobei es sich um modifizierte und unmodifizierte D-Aminosäuren, L-Aminosäuren sowie unnatürliche Aminosäuren handeln kann, bedeuten,
wobei mindestens einer der Reste R1-R10 eine Gruppe -(Y)ₙ-COOM, -(Y)ₙ-COOR13 oder -(Y)ₙ-C(O)NR14R15 ist,
wobei Y -CH₂-, -CH(CH₃)-, -CH₂-CH₂-, -(CH₂)₃-, -CH=CH-, -C≡C-, -O-CH₂-CH₂-, -O-CH₂-CH₂-O-CH₂-CH₂- oder -NH-CH₂-CH₂- darstellt und n = 0 oder 1 ist,
R11 einen C1-C10 Alkylrest bedeutet, und
R12, R13 unabhängig voneinander Wasserstoff oder einen C1-C10-Alkylrest bedeuten.

2. Verbindung gemäß Anspruch 1, wobei in der allgemeinen Formel 1
der Rest R11 eine Ethylgruppe ist,
die Reste R9, R10 Methylgruppen sind,
die Reste R1, R4, R5, R6, R7, R8 Wasserstoff bedeuten,
einer der Reste R2 und R3 Wasserstoff und der andere -(Y)ₙ-COOR13, -(Y)ₙ-COOM mit M = Na, K, [N(R12)₄]⁺ oder -(Y)ₙ-C(O)NR14R15 bedeutet
oder die Reste R2 und R3 unabhänaia voneinander -(Y)ₙ-COOR13, -(Y)ₙ-COOM mit M = Na, K, [N(R12)₄]⁺ oder -(Y)ₙ-C(O)NR14R15 bedeuten.

3. Substrat, umfassend eine Verbindung gemäß Anspruch 1 oder 2, **dadurch gekennzeichnet, dass** die Verbindung über einen Peptidrest oder einen Kohlenwasserstoffrest der Verbindung, optional über eine zusätzliche Linkergruppe, insbesondere eine Peptid-, Polyester-, Polyamid, Kohlenwasserstoff- oder Polyethylenglycol-Linkergruppe, an eine Oberfläche aus Metall, Keramik, Glas oder Polymermaterial, wobei es sich bei dem Polymermaterial vorzugsweise um ein Harz handelt, gebunden ist.

4. Verfahren zur Herstellung einer Verbindung gemäß Anspruch 1 oder 2, **dadurch gekennzeichnet, dass** die Ausgangsverbindungen der Formel 2 und der Formel 3 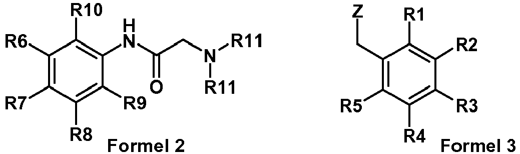 in fester oder gelöster Form miteinander umgesetzt werden, wobei Z in Formel 3 eine substituierbare Gruppe, insbesondere ein Halogenatom oder eine Tosylatgruppe, bedeutet, und die Reste R11 und R1-R10 die in Anspruch 1 oder 2 definierte Bedeutung haben.

5. Verfahren gemäß Anspruch 4, **dadurch gekennzeichnet, dass** das molare Verhältnis der Ausgangsverbindungen der Formel 2 und der Formel 3 1:1 beträgt und/oder die Umsetzung bei einer Temperatur von 20 - 200 °C, vorzugsweise bei 20 - 80 °C, erfolgt und die Umsetzung mit oder ohne Lösungsmittel erfolgt, wobei es sich bei dem optionalen Lösungsmittel um ein organisches Lösungsmittel oder Wasser handelt.

6. Verfahren gemäß Anspruch 4 oder 5, **dadurch gekennzeichnet, dass** die beiden Ausgangsverbindungen der Formel 2 und der Formel 3 in fester Form bei Raumtemperatur (20 bis 30 °C) miteinander vermischt werden und ohne Lösungsmittel umgesetzt werden.

7. Verfahren gemäß Anspruch 4 oder 5, **dadurch gekennzeichnet, dass** die Ausgangsverbindungen der Formel 2 und der Formel 3 in einer Mikrowellen-assistierten Reaktion innerhalb von 1 h miteinander umgesetzt werden, wobei die Mikrowellen-assistierte Reaktion in einem Lösungsmittel durchgeführt wird.

8. Verfahren zur Herstellung eines Substrats gemäß Anspruch 3, **dadurch gekennzeichnet, dass** die Ausgangsverbindungen der Formel 2 und der Formel 3 wie in Anspruch 4 definiert in einem Lösungsmittel miteinander umgesetzt werden, wobei eine der Ausgangsverbindungen der Formel 2 und der Formel 3 Festphasen-gebunden ist und die andere Ausgangsverbindung der Formel 2 und der Formel 3 in Lösung vorliegt, wobei die Festphasen-Bindung über einen der Reste R1-R10, optional über eine zusätzliche Linkergruppe, insbesondere eine Peptid-, Polyester-, Polyamid, Kohlenwasserstoff- oder Polyethylenglycol-Linkergruppe, erreicht wird, und es sich bei der festen Phase um Metall, Keramik, Glas oder ein Polymermaterial handelt, wobei es sich bei dem Polymermaterial vorzugsweise um ein Harz handelt.

9. Verwendung einer Verbindung gemäß einem der Ansprüche 1-2 als Geschmacksstoff, insbesondere als Bitterstoff, mit der Maßgabe, dass es sich nicht um ein Verfahren zur therapeutischen Behandlung des menschlichen oder tierischen Körpers handelt.

10. Verwendung gemäß Anspruch 9 in der Medizin, Biologie, Medizintechnik sowie in der Kosmetik, der pharmazeutischen, chemischen und Nahrungsmittelindustrie.

## Claims

1. A compound of the general formula 1 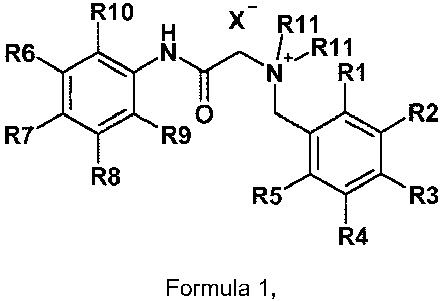 wherein
X⁻ means halogenide, pseudohalogenide, sulfate, benzoate, acetate, trifluoroacetate, hydroxide, saccharinate, or capsaicinate,
R1-R10 independently of each other mean hydrogen, halogen, C1-C5-alkyl-, C1-C4-alkoxy-, C1-C20-alkoxycarbonyl-, -NH-P, -O-P, wherein P is a hydrogen, a peptide residue consisting of 1-30 amino acids, wherein these may be modified and unmodified D-amino acids, L-amino acids as well as unnatural amino acids, -(Y)ₙ-COOR13, -(Y)ₙ-COOM with M = Na, K, [N(R12)₄]⁺,
or -(Y)ₙ-C(O)NR14R15,
wherein R14 and R15 mean hydrogen, a C1-C12-alkyl-, or a peptide residue consisting of 1-30 amino acids, wherein these may be modified and unmodified D-amino acids, L-amino acids as well as unnatural amino acids,
wherein at least one of the residues R1-R10 is a group -(Y)ₙ-COOM, -(Y)ₙ-COOR13, or -(Y)ₙ-C(O)NR14R15,
wherein Y represents -CH₂-, -CH(CH₃)-, -CH₂-CH₂-, -(CH₂)₃-, -CH=CH-, -C≡C-, -O-CH₂-CH₂-, -O-CH₂-CH₂-O-CH₂-CH₂-, or -NH-CH₂-CH₂-, and n = 0 or 1,
R11 means a C1-C10-alkyl residue, and
R12, R13 independently of each other mean hydrogen or a C1-C10-alkyl residue.

2. The compound according to claim 1, wherein in the general formula 1
the residue R11 is an ethyl group,
the residues R9, R10 are methyl groups,
the residues R1, R4, R5, R6, R7, R8 mean hydrogen,
one of the residues R2 and R3 means hydrogen and the
other -(Y)ₙ-COOR13, -(Y)ₙ-COOM with M = Na, K, [N(R12)₄]⁺, or -(Y)ₙ-C(O)NR14R15, or the residues R2 and R3 independently of each other mean -(Y)ₙ-COOR13, -(Y)ₙ-COOM with M = Na, K, [N(R12)₄]⁺, or -(Y)ₙ-C(O)NR14R15.

3. A substrate comprising a compound according to claim 1 or 2, **characterized in that** the compound is bound to a surface made of metal, ceramics, glass, or a polymeric material, wherein the polymeric material preferably is a resin, via a peptide residue or a hydrocarbon residue of the compound, optionally via an additional linker group, particularly a peptide, polyester, polyamide, hydrocarbon, or polyethylene glycol linker group.

4. A method for the production of a compound according to claim 1 or 2, **characterized in that** the starting compounds of formula 2 and of formula 3 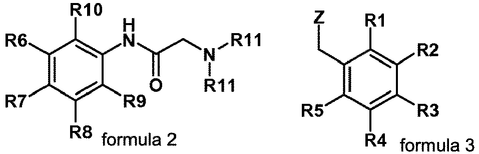 are reacted with each other in a solid or dissolved form, wherein Z in formula 3 means a substitutable group, particularly a halogen atom or a tosylate group, and the residues R11 and R1-R10 have the meaning defined in claim 1 or 2.

5. The method according to claim 4, **characterized in that** the molar ratio of the starting compounds of formula 2 and of formula 3 is 1:1 and/or the reaction takes place at a temperature of 20-200°C, preferably at 20-80°C, and the reaction takes place with or without a solvent, wherein the optional solvent is an organic solvent or water.

6. The method according to claim 4 or 5, **characterized in that** the two starting compounds of formula 2 and of formula 3 are mixed together in a solid form at room temperature (20 to 30°C) and are reacted without a solvent.

7. The method according to claim 4 or 5, **characterized in that** the starting compounds of formula 2 and of formula 3 are reacted with each other in a microwave-assisted reaction within 1h, wherein the microwave-assisted reaction is carried out in a solvent.

8. The method for the production of a substrate according to claim 3, **characterized in that** the starting compounds of formula 2 and of formula 3 as defined in claim 4 are reacted with each other in a solvent, wherein one of the starting compounds of formula 2 and of formula 3 is solid phase-bound and the other starting compound of formula 2 and of formula 3 is present in solution, wherein the solid phase-bond is achieved via one of the residues R1-R10, optionally via an additional linker group, particularly a peptide, polyester, polyamide, hydrocarbon, or polyethylene glycol linker group, and the solid phase is metal, ceramics, glass, or a polymeric material, wherein the polymeric material preferably is a resin.

9. Use of a compound according to any one of claims 1-2 as a flavoring agent, particularly as a bitter principle, provided that it is not a method for the therapeutic treatment of the human or animal body.

10. Use according to claim 9 in medicine, biology, medical engineering as well as in cosmetics, the pharmaceutical, chemical, and food industry.

## Revendications

1. Composé de formule générale 1 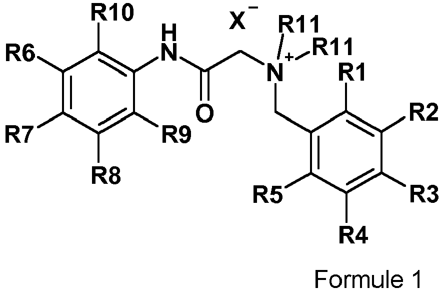 dans laquelle
X⁻ représente un halogénure, un pseudohalogénure, un sulfate, un benzoate, un acétate, un trifluoroacétate, un hydroxyde, un saccharinate ou un capsaicinate,
R1 à R10 représentent indépendamment les uns des autres un hydrogène, un halogène, un alkyle en C₁ à C₅, un alcoxy en C₁ à C₄, un alcoxycarbonyle en C₁ à C₂₀, -NH-P, -O-P, où P est un hydrogène, un radical peptidique composé de 1 à 30 acides aminés, pouvant être des acides aminés D modifiés et non modifiés, des acides aminés L, ainsi que des acides aminés non naturels, -(Y)ₙ-COOR13, -(Y)ₙ-COOM avec M = Na, K, [N(R12)₄]⁺ ou -(Y)ₙC(O)NR14R15,
dans laquelle R14 et R15 représentent un hydrogène, un alkyle en C₁ à C₁₂ ou un radical peptidique composé de 1 à 30 acides aminés, pouvant être des acides aminés D modifiés et non modifiés, des acides aminés L et des acides aminés non naturels,
dans laquelle au moins l'un des radicaux R1 à R10 représente un groupe -(Y)ₙ-COOM, -(Y)ₙ-COOR13 ou -(Y)ₙC(O)NR14R15,
dans laquelle Y représente -CH₂-, -CH(CH₃)-, -CH₂-CH₂-, -(CH₂)₃-, -CH=CH-, -C≡C-, -O-CH₂-CH₂-, -O-CH₂-CH₂-O-CH₂-CH₂- ou -NH-CH₂-CH₂- et n = 0 ou 1,
R11 représente un radical alkyle en C₁ à C₁₀, et
R12, R13 représentent indépendamment l'un de l'autre un hydrogène ou un radical alkyle en C₁ à C₁₀.

2. Composé selon la revendication 1, dans lequel dans la formule générale 1
le radical R11 représente un groupe éthyle,
les radicaux R9, R10 représentent des groupes méthyle,
les radicaux R1, R4, R5, R6, R7, R8 représentent un hydrogène,
l'un des radicaux R2 et R3 représente un hydrogène et l'autre -(Y)ₙ-COOR13, -(Y)ₙ-COOM avec M = Na, K, [N(R12)₄]⁺ ou -(Y)ₙC(O)NR14R15,
ou les radicaux R2 et R3 représentent indépendamment l'un de l'autre -(Y)ₙ-COOR13, -(Y)ₙ-COOM avec M = Na, K, [N(R12)₄]⁺ ou -(Y)ₙC(O)NR14R15.

3. Substrat comprenant un composé selon la revendication 1 ou 2, **caractérisé en ce que** le composé est lié, via un radical peptidique ou un radical hydrocarboné du composé, facultativement via un groupe de liaison supplémentaire, en particulier un groupe de liaison peptide, polyester, polyamide, hydrocarbure ou polyéthylène glycol, à une surface en métal, céramique, verre ou matériau polymère, dans lequel le matériau polymère est de préférence une résine.

4. Procédé de préparation d'un composé selon la revendication 1 ou 2, **caractérisé en ce que** les composés de départ de formule 2 et de formule 3 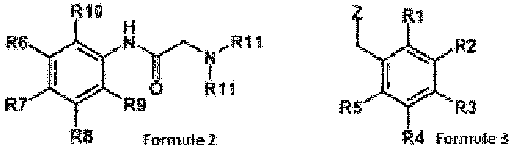 réagissent l'un avec l'autre sous une forme solide ou dissoute, dans lequel Z dans la formule 3 représente un groupe substituable, en particulier un atome d'halogène ou un groupe tosylate, et les radicaux R11 et R1 à R10 sont tels que définis selon revendication 1 ou 2.

5. Procédé selon la revendication 4, **caractérisé en ce que** le rapport molaire des composés de départ de formule 2 et de formule 3 est de 1:1 et/ou la réaction a lieu à une température allant de 20 à 200 °C, de préférence de 20 à 80 °C et la réaction est mise en œuvre avec ou sans solvant, dans lequel le solvant facultatif est un solvant organique ou de l'eau.

6. Procédé selon la revendication 4 ou 5, **caractérisé en ce que** les deux composés de départ de formule 2 et de formule 3 sont mélangés sous une forme solide à température ambiante (20 à 30 °C) et mis en réaction sans solvant.

7. Procédé selon la revendication 4 ou 5, **caractérisé en ce que** les composés de départ de formule 2 et de formule 3 sont mis en réaction l'un avec l'autre dans une réaction assistée par micro-ondes en 1 h, dans lequel la réaction assistée par micro-ondes est mis en œuvre dans un solvant.

8. Procédé de production d'un substrat selon la revendication 3, **caractérisé en ce que** les composés de départ de formule 2 et de formule 3 tels que définis selon la revendication 4 sont mis en réaction l'un avec l'autre dans un solvant, dans lequel un premier des composés de départ de formule 2 et de formule 3 est lié à une phase solide et l'autre composé de départ de formule 2 et de formule 3 est en solution, dans lequel la liaison à une phase solide est obtenue via l'un des radicaux R1 à R10, facultativement via un groupe de liaison supplémentaire, en particulier un groupe de liaison peptide, polyester, polyamide, hydrocarbure ou polyéthylène glycol, et la phase solide est en métal, céramique, verre ou en un matériau polymère, dans lequel le matériau polymère est de préférence une résine.

9. Utilisation d'un composé selon l'une quelconque des revendications 1 à 2 comme agent aromatisant, en particulier comme agent amérisant, à condition qu'il ne s'agisse pas d'un procédé de traitement thérapeutique du corps humain ou animal.

10. Utilisation selon la revendication 9 en médecine, biologie, technologie médicale, ainsi qu'en cosmétique, industrie pharmaceutique, chimique et agro-alimentaire.
